# EUROPÄISCHE PATENTSCHRIFT

(11) **EP 1 894 621 B1**
(45) Veröffentlichungstag und Bekanntmachung des Hinweises auf die Patenterteilung: **03.04.2013**
(21) Anmeldenummer: 07112223.8
(22) Anmeldetag: 11.07.2007
(51) Int. Cl.: B01J 21/12, B01J 23/02, B01J 37/02, C07C 1/20, C07C 29/00, B01J 29/03, B01J 29/08, B01J 29/40, B01J 35/10, C07C 11/09

(54) **Verfahren zur Herstellung eines Katalysators zur Gasphasenspaltung von Methyl-tert.-butylether**
Method for producing a catalyst for the gas phase decomposition of methyl tertiary butyl ether
Procédé de préparation d'un catalyseur pour la décomposition en phase gazeuse du méthyl tert-butyl éther

(30) Priorität: 29.08.2006 DE 102006040432
(43) Veröffentlichungstag der Anmeldung: 05.03.2008
(60) Teilanmeldung: 12196240.1
(73) Patentinhaber: Evonik Oxeno GmbH, 45772 Marl (DE)
(72) Erfinder: Zanthoff, Horst-Werner, 45481 Mülheim a.d. Ruhr (DE); Maschmeyer, Dietrich, 45657 Recklinghausen (DE); Quandt, Thomas, 45772 Marl (DE); Nierlich, Franz, 45768 Marl (DE); Santiago Fernandez, Silvia, 33009 Oviedo (ES); Houbrechts, Stephan, B-2570 Duffel (BE); Skillas, Georg, 63456 Hanau (DE); Gaudschun, Kurt-Alfred, 45665 Recklinghausen (DE)

(56) Entgegenhaltungen:
- EP-A- 0 589 557
- GB-A- 1 249 079
- GB-A- 1 355 060
- US-A- 2 744 057
- US-A- 2 861 945
- US-A- 3 140 249
- US-A- 4 079 019
- US-A- 4 238 361
- US-A- 4 239 651
- US-A- 5 171 920
- US-A- 5 254 785
- DATABASE WPI Week 199415 Derwent Publications Ltd., London, GB; AN 1994-124093 XP002463257 & JP 06 072904 A (MITSUBISHI RAYON CO LTD) 15. März 1994 (1994-03-15)

## Beschreibung

Die vorliegende Erfindung betrifft ein Verfahren zur Herstellung eines Katalysators Casphasenspaltung NON MTBE(Methyl-tert.-butylether) in Isobuten und Methanol.

Isoolefine, wie z. B. Isobuten, sind wichtige Zwischenprodukte für die Herstellung einer Vielzahl organischer Verbindungen. Isobuten ist beispielsweise Ausgangsstoff für die Herstellung von Butylkautschuk, Polyisobutylen, Isobuten-Oligomeren, verzweigten C₅-Aldehyden, C₅-Carbonsäuren, C₅-Alkoholen und C₅-Olefinen. Weiterhin wird es als Alkylierungsmittel, insbesondere zur Synthese von tert.-Butylaromaten, und als Zwischenprodukt für die Erzeugung von Peroxiden eingesetzt. Darüber hinaus kann Isobuten als Vorstufe für Methacrylsäure und deren Estern verwendet werden.

In technischen Strömen liegen Isoolefine meist zusammen mit anderen Olefinen und gesättigten Kohlenwasserstoffen mit gleicher Kohlenstoffatomzahl vor. Aus diesen Gemischen sind die Isoolefine allein mit physikalischen Trennmethoden wirtschaftlich nicht abtrennbar.

Beispielsweise liegt Isobuten in üblichen technischen Strömen zusammen mit gesättigten und ungesättigten C₄-Kohlenwasserstoffen vor. Aus diesen Gemischen kann Isobuten wegen der geringen Siedepunktsdifferenz bzw. des geringen Trennfaktors zwischen Isobuten und 1-Buten durch Destillation nicht wirtschaftlich abgetrennt werden. Daher wird Isobuten aus technischen Kohlenwasserstoffen häufig dadurch gewonnen, dass Isobuten zu einem Derivat umgesetzt wird, das sich leicht vom übrigen Kohlenwasserstoffgemisch abtrennen lässt, und dass das isolierte Derivat zu Isobuten und Derivatisierungsmittel zurückgespaltet wird.

Üblicherweise wird Isobuten aus C₄-Schnitten, beispielsweise der C₄-Fraktion eines Steamcrackers, wie folgt abgetrennt. Nach Entfernung des größten Teils der mehrfach ungesättigten Kohlenwasserstoffe, hauptsächlich des Butadiens, durch Extraktion(-sdestillation) oder Selektivhydrierung zu linearen Butenen wird das verbleibende Gemisch (Raffinat I oder selektiv hydriertes Crack-C₄) mit Alkohol oder Wasser umgesetzt. Bei der Verwendung von Methanol entsteht aus Isobuten Methyl-tert.-butylether (MTBE), bei der Verwendung von Ethanol Ethyl-tert.-butylether (ETBE) und bei Einsatz von Wasser tert.-Butanol (TBA). Nach ihrer Abtrennung können diese Derivate in Umkehrung ihrer Bildung zu Isobuten gespalten werden.

Die Spaltung von Alkyl-tert.-butylethern (ATBE) zu den entsprechenden Isoolefinen und Alkoholen sowie die Spaltung von tertiären Alkoholen zu den entsprechenden Isoolefinen und Wasser kann in Gegenwart saurer oder basischer Katalysatoren in der Flüssigphase bzw. Gas/Flüssig-Mischphase oder in der reinen Gasphase durchgeführt werden.

Die Spaltung in der Flüssigphase bzw. Gas/Flüssigphase hat den Nachteil, dass die entstehenden Produkte, gelöst in der Flüssigphase, leichter Nebenreaktionen eingehen können. Beispielsweise kann das bei der Spaltung von MTBE entstehende Isobuten durch sauer katalysierte Dimerisierung oder Oligomerisierung unerwünschte C₈- und C₁₂-Komponenten bilden. Bei den unerwünschten C₈-Komponenten handelt es sich hauptsächlich um 2,4,4-Trimethyl-1-penten und 2,4,4-Trimethyl-2-penten. Darüber hinaus setzt sich besonders an basischen Katalysatoren, ein Teil des bei der Spaltung entstehenden Methanols unter Wasserabspaltung zu Dimethylether um. Wird die Reaktion nicht unter Drücken oberhalb des Sättigungsdampfdruckes des Reaktionsgemisches durchgeführt, um diesen Problemen entgegenzuwirken, so ist ein zusätzliches Lösemittel notwendig.

In der Gasphase kann die Bildung von Nebenprodukten durch Reaktion der Spaltprodukte mit sich selbst aufgrund deren geringerer Konzentrationen im Vergleich zur Spaltung in der Flüssigphase zurückgedrängt werden. Es können allerdings wegen der höheren Spalttemperaturen andere Nebenreaktionen auftreten. Bei der Gasphasenspaltung sind daher Katalysatoren erforderlich, die mit sehr hoher Selektivität die Spaltung von tertiären Alkylethern oder tertiären Alkoholen in Isoolefin sowie Alkohol oder Wasser katalysieren, jedoch keine Nebenreaktionen, wie beispielsweise C-C-Spaltung oder Dehydrierung sowie C-C-Kupplungsreaktionen oder Etherbildung der entstehenden Alkohole, begünstigen. Weiterhin sollen diese Katalysatoren hohe Raum-Zeit-Ausbeuten ermöglichen und eine hohe Standzeit aufweisen. Darüber hinaus ist eine Spaltung des Edukts mit möglichst hoher Selektivität für das entstehende Isoolefin bei einem Druck größer 0,3 MPa wünschenswert.

Als Katalysatoren für die Gasphasenspaltung von Alkyl-tert.-alkylethern (ATAE) und tertiären Alkoholen zu den entsprechenden Isoolefinen sowie Alkohol oder Wasser sind in der Literatur eine Vielzahl von Katalysatoren beschrieben. Dies gilt insbesondere für Katalysatoren, die für die Spaltung von Methyl-tert.-butylether (MTBE) genutzt werden.

Als Katalysatoren werden dabei meistens Metalloxide mit einer Summenformel MₐOₓ, Metallmischoxide mit Summenformeln MₐM_{b}MₙO_{y}, insbesondere solche, die M = Si oder M =Al enthalten, Säuren auf Metalloxidträgern oder Metallsalze verwendet.

In US 4,254,290 werden als Spaltkatalysatoren beispielsweise SiO₂/Al₂O₃ oder WO₃/Al₂O₃ beschrieben. In US 4,320,232 und US 4,521,638 werden zur Spaltung von tertiären Ethern Katalysatoren, bestehend aus Phosphorsäure auf Trägern, beansprucht. Aluminiumoxid auf Kieselgel wird in US 4,398,051 als Spaltkatalysator genutzt. Für den gleichen Zweck werden in den beiden Patentschriften US 4,357,147 und US 5,254,785 Zeolithe verwendet.

In JP 59010528 wird als Spaltkatalysator sulfatiertes Titandioxid oder Zirkondioxid verwendet. In US 5,607,992 wird zur Spaltung von Ethern ein Zirkoniumoxid/Ceroxid-Katalysator, in US 6,124,232 Zirkoniumoxid/Wolframoxid, in US 6,162,757 ein Mischoxid von Zirkonium und Seltenen Erden eingesetzt.

In WO 2005-066101 wird ein Katalysator mit allgemeinen Summenformel XₙYₙZₚO_{q} beansprucht, wobei X mindestens ein Element der vierten Gruppe des Periodensystems der Elemente, Y mindestens ein Metall aus der dritten und/oder sechsten Gruppe und Z mindestens ein Element aus der siebten, achten oder elften Gruppe ist.

In JP 5-229965 (1993) wird ein Katalysator mit der Summenformel SiₐX_{b}Y_{c}Z_{d}Oₑ beansprucht. (Hier stehen Si und O jeweils für Silizium und Sauerstoff; X ist zumindest ein Element, das aus der Gruppe, bestehend aus Titan und Zirkonium, ausgewählt ist; Y ist ein Element, das aus der Gruppe, bestehend aus Magnesium und Calcium ausgewählt ist; Z ist zumindest ein Element, das aus der Gruppe, bestehend aus Natrium, Kalium, Chlor und Schwefel ausgewählt ist; a, b, c, d und e zeigen das Atomverhältnis der einzelnen Elemente an. Wenn a = 1 ist, dann sind b =0,001 bis 10, c =0,0001 bis 5, d =0 bis 1; e ist die notwendige Sauerstoffatomzahl, um den Atomwert der vorstehend genannten einzelnen Bestandteile zu erfüllen)

In EP 0 589 557 wird u. a. ein Katalysator für die MTBE-Spaltung beansprucht, der formal aus Magnesiumoxid, Aluminiumoxid und Siliziumdioxid besteht. Bei seiner Herstellung wird in einem ersten Schritt ein Alumosilikat mit einer wässrigen Magnesiumsalzlösung in der Weise imprägniert, das während der Imprägnierung der pH-Wert der Tränklösung durch Zugabe einer Base auf einen pH-Wert von 7 bis 11 eingestellt werden kann. Um besonders aktive und selektive Katalysatoren zu erhalten, sind zum Teil Imprägnierzeiten von über 200 h erforderlich. Der Katalysator liegt in pulverisierter Form vor.

Aus US-A-3 140 249 ist ein Verfahren bekannt, bei dem ein Alumosilikat mit einer sauren Lösung von beispielsweise CaCl₂ behandelt und kalziniert wird. Der auf diese Weise hergestellte Katalysator ist für das katalytische Cracken von schweren MineralölFraktionen in Leichtbenzine bestimmt. Er wird somit in einem katalytischen Cracker einer Erdöl-Raffinerie eingesetzt. Der Einsatz dieses Katalysators in der Petro-Folgechemie, insbesondere für das Spalten von MTBE in Isobuten und Methanol, wird nicht offenbart.

US-A-4 239 651 beschreibt ein Verfahren, bei dem ein Siliziumoxid-Aluminiumoxid-Gel mit einer Erdalkalimetallsalzlösung bei einem pH-Wert von 5,5 oder kleiner behandelt wird. Weiter zeigt dieses Dokument eine Dampfbehandlung bei 760°C. Auch dieser Katalysator ist für den Einsatz in einem katalytischen Cracker für Mineralöl bestimmt. Die Spaltung von MTBE wird nicht beschrieben. Zur Darreichungsform des Katalysators gibt das Dokument keine Auskunft.

US-A-2 744 057 beschreibt ein Verfahren, bei dem ein SiO₂-Al₂O₃-Träger mit Barium, Magnesium, Calcium oder Strontium ausgetauscht und kalziniert wird. Dabei liegen zumindest am Ende der Behandlung Bedingungen vor, bei denen eine Erdalkalimetallsalzlösung anwesend und der pH-Wert kleiner 7 ist. Auch dieser Katalysator ist für den Einsatz in einem katalytischen Erdöl-Cracker in einem Temperaturbereich von 370°C bis 590°C bestimmt. Die Gasphasen-Spaltung von MTBE wird in diesem Dokument nicht thematisiert. Die Darreichungsform des Katalysators ist nicht weiter beschrieben.

GB-A-1 355 060 beschreibt ebenfalls einen Katalysator zum Spalten von Benzin-Fraktionen. Bei dessen Herstellung wird ein Zeolith vom Typ Z-14US mit einer sauren Lösung aus Nickel und Magnesium-Kationen behandelt und kalziniert. Die Katalysatoren werden zu partikelförmigen Produkten weiter verarbeitet, um sie in katalytischen Anwendungen nutzbar zu machen. Im Zuge dessen werden aus dem Katalysator vergleichsweise große Partikel mit einem Durchmesser im Bereich von etwa 1,6 bis 6 mm geformt, um sie in Wanderbetten von katalytischen Crackern einsetzen zu können. Des Weiteren wird beschrieben, dass die Zeolithe in Form von mikroskopischen Kugeln aufgearbeitet werden können, deren Durchmesser im Bereich von 50 bis 200 µm liegt und die für den Einsatz in Wirbelschicht-katalytischen Crackern bestimmt sind. Eine Verarbeitung der Zeolithe in Tablettenform ist nicht beschrieben. Die Kalzinierung der Zeolithe findet vor ihrer Weiterverarbeitung zu Partikel bzw. MikroKugeln statt.

US 4 079 019 A befasst sich mit der Herstellung von Katalysator-Partikel für die Umsetzung von Kohlenwasserstoffen, bei dem eine Alkalimetall-Silikat Lösung mit einer Aluminiumsalz-Lösung vermischt wird um eine wässrigen Slurry eines Alumosilikat-Hydrogels zu erhalten, bei welchem der pH-Wert der Slurry durch Zugabe von Magnesiumoxid oder Calciumoxid auf 5 bis 7 eingestellt wird und bei dem kristalline Alumosilikat-Zeolithe in die Lösung eingegeben werden. Der kristalline Katalysator wird durch Ofentrocknung entweder zu Pulver verarbeitet oder sprühgetrocknet, sodass Mikrokügeln mit einem Durchmesser von 75 µm anfallen. Als weitere Darreichungsform des Katalysators sind Pillen oder Extrudate erwähnt. Hinsichtlich deren Herstellung verweist das Dokument auf den allgemeinen Stand der Technik.

GB 1 249 079 liegt auf dem Gebiet der katalytischen Epoxidierung von Olefinen. Der zugehörige, Titanhaltige Katalysator wird durch Behandlung eines Alumosilikat-Trägers mit einer Mischung enthaltend Titantetrachlorid und Magnesiumnitrat-Hexahydrat behandelt, bei 160°C getrocknet und sodann bei 800°C kalziniert. Der erhaltene Katalysator enthält 2.2 Gew% Ti und 1.1 Gew-% Mg auf dem Alumosililkat-Träger. Die Darreichungsform des Katalysators wird nicht vertieft.

US-A-5 254 785 befasst sich mit der Spaltung von MTBE in Isobuten und Methanol. Bei der Herstellung des hierzu verwendeten Katalysators werden Zeolith-Pellets mit einem Anteil von 20% Aluminiumoxid mit einer wässrigen Lösung aus Kalziumchlorid behandelt. Sodann werden die Zeolithe mit hochreinem Wasser (pH 6,7) gewaschen bis keine Chloridionen mehr nachweisbar sind. Sodann wird der Zeolith bei einer Temperatur von 100°C für acht Stunden im Vakuum getrocknet. Eine weitergehende Wärmebehandlung der pelletierten und behandelten Zeolith-Pellets findet nicht statt.

JP 06 072904 A beschreibt die katalytische Spaltung von MTBE an einem Katalysator, der Siliziumaluminiumzirkon und mindestens ein Element enthält welches ausgewählt ist von Natrium, Kalium, Cäsium, Cerium, Zink, Magnesium und Kalzium in oxidischer Form. Der Katalysator wird nach der Wärmebehandlung mit Aluminiumsulfat behandelt und einer weiteren Wärmebehandlung im Bereich von 500 bis 1100°C unterworfen.

US 4 238 361 A offenbart ein Verfahren, bei dem ein Aluminiumoxid-Siliziumoxid Cogel bei einem pH-Wert von 4 mit einer Magnesiumnitrat-Lösung behandelt wird. Der Katalysator wird vor dem katalytischen Test kalziniert. Der Katalysator wird als Spaltkatalysator eingesetzt, beispielsweise zum Hydrocracking, zum Hydroforming und zur Hydroentschwefelung. Sein Einsatz in der Gasphasenspaltung von MTBE ist nicht beschrieben.

US 2 861 945 offenbart ein Verfahren zur Herstellung eines Katalysators, der zu 40% Aluminiumoxid und im Rest Siliziumoxid enthält. Nach dem Waschen wird der Katalysator bei 204°C getrocknet und bei 538°C kalziniert. Sodann wird der Katalysator mit Kalziumhydroxid bzw. mit Magnesiumnitratlösungen behandelt. Es wird vorgeschlagen, den Katalysator fein verteilt oder pulverförmig in fluidkatalytischen Crackern einzusetzen oder granuliert oder in Pillenform in Festbetten oder Wanderbetten von katalytischen Crackern. Die Herstellung des Granulats oder der Pillen wird nicht weiter vertieft. Als Einsatzzweck des Katalysators wird das katalytische Cracken von Öl bei der Benzinherstellung genannt.

In US 5,171,920 wird im Beispiel 4 die Herstellung eines Spaltkatalysators beschrieben, der formal die Komponenten Siliziumdioxid, Aluminiumoxid und Magnesiumoxid enthält. Die Herstellung geschieht derart, dass zunächst Siliziumdioxid mit einer wässrigen Magnesiumnitrat-Lösung getränkt/imprägniert wird, nach einer Zwischentrocknung erfolgt eine weitere Tränkung/Imprägnierung mit einer wässrigen Aluminiumnitrat-Lösung. Anschließend wird nach Vortrocknung kalziniert.

Die bekannten Katalysatoren weisen bei der Spaltung von Alkyl-tert.-alkylethern oder tertiären Alkoholen in Isoolefin sowie Alkohol einen oder mehrere der folgenden Nachteile aus: geringe Selektivität hinsichtlich der Zielprodukte, Anwendung hoher Temperaturen bei der Spaltung, zum Teil über 500 °C, geringe Standzeiten der Katalysatoren sowie aufwändige und damit kostenintensive Herstellung des Katalysators.

Es bestand daher die Aufgabe, einen Spaltkatalysator bereitzustellen, der einen oder mehrere dieser Nachteile nicht aufweist.

Überraschenderweise wurde nun gefunden, dass Mischoxidkatalysatoren, die formal aus Aluminiumoxid, Siliziumdioxid und zumindest einem Oxid aus der Gruppe der Alkalimetalloxide, und Magnesiumoxid bestehen und die vorzugsweise durch mindestens einmaliges Tränken eines Alumosilikats mit einer sauren wässrigen Lösung, die ein Magnesiumsalz oder enthält, und anschließender Kalzination hergestellt werden, Alkyl-tert.-Alkylether (ATAE) und tertiäre Alkohole mit sehr hohen Selektivitäten zu den entsprechenden Isoolefinen spalten und sehr hohe Standzeiten aufweisen. Das Tränken kann z. B. durch ein einfaches Vermischen des Alumosilikats mit der sauren wässrigen Tränklösung erfolgen, ohne dass eine zusätzliche pH-Kontrolle nötig ist und ohne dass besonders lange Tränkzeiten notwendig sind. Dieser Befund ist überraschend, da in EP 0 589 557 A2 ausdrücklich darauf hingewiesen wird, dass während der Tränkung der pH-Wert in der überstehenden Lösung durch Zusatz von Basen im Bereich von 7 bis 11 gehalten werden muss und dass lange Tränkzeiten erforderlich sind, um Katalysatoren mit hoher Aktivität und geringer Neigung zur Bildung von Nebenprodukten zu erhalten.

Gegenstand der vorliegenden Erfindung ist deshalb ein Verfahren nach Anspruch 1.

Die Herstellung von Isoolefinen durch Gasphasenspaltung von ATAE oder tertiären Alkoholen unter Verwendung des erfindungsgemäß hergestellten Katalysators hat mehrere Vorteile: Auch bei Umsätzen der Einsatzstoffe von über 70 % entstehen die entsprechenden Isoolefine in Selektivitäten von über 99 %. Bei Spaltung von ATBE liegen die Selektivitäten für die aus dem abgespalteten Alkohol gebildeten Ethern unter 30 %. Die Spaltung kann bei für Spaltreaktionen relativen niedrigen Temperaturen von 150 bis 450 °C, bevorzugt bei Temperaturen von 230 bis 350 °C durchgeführt werden. Die Umsetzungen kann bei Drücken von größer als 0,3 MPa durchgeführt werden, sodass eine Kondensation der gebildeten Isoolefine gegen Kühlwasser möglich ist. Der Katalysator zeichnet sich durch eine hohe Standdauer aus. Der Katalysator enthält keine Schwermetalle, sodass weder bei seiner Herstellung noch bei seiner Entsorgung ökologisch bedenkliche Stoffe anfallen. Durch Variation des Anteils an Erdalkalimetalloxid kann die Aktivität für jedes Edukt optimal eingestellt werden.

Das Verfahren sowie die Katalysatoren werden nachfolgend beispielhaft beschrieben.

Unter Alumosilikaten sollen im Rahmen der vorliegenden Erfindung Verbindungen verstanden werden, die sich formal im Wesentlichen aus Anteilen von Aluminiumoxid (Al₂O₃) und Siliziumdioxid (SiO₂) zusammensetzen. Die im erfindungsgemäßen Verfahren eingesetzten Alumosilikate können von 1 bis 99 Massen-% an Siliziumdioxid und von 1 bis 99 Massen-% an Aluminiumoxid (Al₂O₃) aufweisen. Die erfindungsgemäßen Alumosilikate können aber auch noch geringe Anteile an Alkali- oder Erdalkalimetalloxiden enthalten. In dem erfindungsgemäßen Verfahren können als Alumosilikate auch Zeolithe wie z. B. Zeolith A, X, Y, USY oder ZSM-5 oder amorphe Zeolithe (wie beispielsweise MCM 41 von Mobil Oil) eingesetzt werden. Die Bestimmung der Zusammensetzung der eingesetzten Alumosilikate bzw. der erhaltenen Katalysatoren kann z. B. durch klassische Analyse, Schmelzaufschluss mit Borax und RFA (Röntgenfluoreszenzanalyse), energiedispersive Röntgenanalyse, Flammenspektroskopie (A1 und Mg, nicht Si), nassem Aufschluss und anschließender ICP-OES (Optische Emissionsspektrometrie mit induktiv gekoppelten Hochfrequenzplasma) oder Atomabsorptionsspektroskopie erfolgen. Vorzugsweise erfolgt die Bestimmung der Zusammensetzung durch Schmelzaufschluss mit Borax und anschließende RFA oder durch nassen Aufschluss und anschließender ICP-OES.

Die im erfindungsgemäßen Verfahren eingesetzten Alumosilikate können amorph oder kristallin sein. Geeignete kommerzielle Alumosilikate die als Ausgangsstoffe im erfmdungsgemäßen Verfahren eingesetzt werden können sind beispielsweise Alumosilikate, die durch Fällung, Gelierung oder Pyrolyse hergestellt wurden.

Vorzugsweise werden in dem erfindungsgemäßen Verfahren Alumosilikate eingesetzt, die von 5 bis 40 Massen-%, bevorzugt 10 bis 35 Massen-% Aluminiumoxid und von 60 bis 95 Massen-%, bevorzugt von 65 bis 90 Massen-% Siliziumdioxid aufweisen (bezogen auf die Trockenmasse; Behandlung: Glühen bei 850 °C für 1 h). Ein besonders bevorzugtes Alumosilikat, welches in dem erfindungsgemäßen Verfahren eingesetzt werden kann, weist einen formalen Anteil an Al₂O₃ von 10 bis 15 Massen-% und einen Anteil an Siliziumdioxid von 73 bis 78 Massen-% auf. Ein solches Alumosilikat wird von der Firma Grace Davison, unter der Bezeichnung Davicat O 701 angeboten.

Das Alumosilikat kann in den unterschiedlichsten Formen in dem erfindungsgemäßen Verfahren eingesetzt werden. So kann das Alumosilikat in Form von Formkörpern, wie z. B. Tabletten, Pellets, Granulat, Strängen oder Extrudaten eingesetzt werden. Das Alumosilikat kann aber auch als Alumosilikatpulver eingesetzt werden. Als Ausgangsmaterial kann von Pulvern mit unterschiedlicher mittlerer Korngröße und unterschiedlicher Korngrößenverteilung ausgegangen werden. Bevorzugt wird in dem erfindungsgemäßen Verfahren ein Alumosilikatpulver eingesetzt, bei dem 95 % der Partikel eine mittlere Korngröße von 5 bis 100 µm, vorzugsweise 10 bis 30 µm und besonders bevorzugt 20 bis 30 µm aufweisen. Die Bestimmung der Korngröße kann z. B. durch Laserbeugung mit einem Partikelanalysator der Firma Malvern, wie z. B. dem Mastersizer 2000 durchgeführt werden.

### Verfahrensschritt a)

In Schritt a) wird eine Magnesiumsalzlösung eingesetzt. Es ist auch möglich in Schritt a) eine Salzlösung einzusetzen, die ein oder mehrere Salze von Alkalimetallen und von Magnesium aufweist. Zur Herstellung der wässrigen Salzlösungen können Alkali- und/oder Magnesium verbindungen verwendet werden, die wasserlöslich sind oder durch Zugabe einer Säure in wasserlösliche Verbindungen übergehen. Vorzugsweise werden als Salze die Nitrate der der Alkali- bzw. Magnesium eingesetzt.

Die eingesetzte saure wässrige Alkali- und/oder Magnesiumsalzlösung weist vorzugsweise einen pH-Wert von kleiner 6, bevorzugt von kleiner 6 bis 3, und besonders bevorzugt von 5,5 bis 3,5 auf. Die Bestimmung des pH-Werts kann z. B. mit Hilfe einer Glaselektrode oder Indikatorpapier durchgeführt werden. Weist die Salzlösung einen pH-Wert auf, der größer oder gleich 6 ist, so kann der pH-Wert durch Zugabe einer Säure, vorzugsweise der Säure dessen Alkali- und/oder Magnesiumsalz in der Lösung vorhanden ist, eingestellt werden. Vorzugsweise wird, wenn die Alkali- und/oder Magnesiumsalzlösung als Salze die Nitrate aufweist, als Säure Salpetersäure eingesetzt.

In dem erfmdungsgemäßen Verfahren wird eine Magnesium- salzlösung eingesetzt. Bevorzugt werden Magnesiumsalzlösungen eingesetzt, die als Magnesiumsalze die Salze starker Mineralsäuren, wie beispielsweise Magnesiumnitrat-Hexahydrat oder Magnesiumsulfat-Heptahydrat aufweisen. Bevorzugt wird in dem erfindungsgemäßen Verfahren eine Magnesiumsalzlösung und besonders bevorzugt eine Magnesiumnitrat-(Hexahydrat)-Lösung eingesetzt.

Die in Schritt a) eingesetzte Alkali- und/oder Magnesium salzlösung weist ein Magnesiumsalz auf; der Magnesiumgehalt der Lösung beträgt vorzugsweise von 0,1 bis 3 Mol/l, bevorzugt von 0,5 bis 2,5 Mol/l.

Das Behandeln in Schritt a) kann auf verschiedene. Weisen erfolgen, die geeignet sind, das Alumosilikat mit der Alkali- und/oder Magnesiumsalzlösung in Kontakt zu bringen. Mögliche Behandlungsmethoden sind z. B. Imprägnieren, Tränken, Besprühen oder Begießen des Alumosilikats mit der Alkali- und/oder Magnesiumsalzlösung. Es kann vorteilhaft sein, wenn das Behandeln des Alumosilikats so erfolgt, dass die Alkali- und/oder Magnesiumsalzlösung zumindest 0,1 bis 5 h bevorzugt von 0,5 bis 2 h auf das Alumosilikat einwirken kann. Eine solche Einwirkzeit kann insbesondere dann vorteilhaft sein, wenn das Behandeln durch einfaches Tränken erfolgt.

In einer bevorzugten Ausführungsform des erfindungsgemäßen Schrittes a) des erfindungsgemäßen Verfahrens kann die Behandlung von Alumosilikat, insbesondere Alumosilikatformkörpern mit der Alkali- und/oder Magnesiumsalzlösung beispielsweise durch Vakuumimprägnierung in einer dafür geeigneten Vakuumimprägnieranlage erfolgen. Bei dieser Art der Behandlung wird das Alumosilikat in der Vakuumimprägnieranlage zunächst evakuiert. Anschließend wird die Alkali- und/oder Magnesiumsalzlösung bis über den oberen Rand der Trägerschüttung gesaugt, so dass das gesamte Alumosilikat mit der Lösung bedeckt ist. Nach einer Einwirkzeit, die vorzugsweise von 0,1 bis 10 h, bevorzugt von 0,5 bis 2 h beträgt, wird die Lösung, die nicht vom Träger aufgenommen wurde, abgelassen.

In einer weiteren bevorzugten Ausführungsform des erfindungsgemäßen Schrittes a) des erfindungsgemäßen Verfahrens kann die Behandlung von Alumosilikat, insbesondere Alumosilikatformkörpern mit der Alkali- und/oder Magnesiumsalzlösung beispielsweise Besprühen oder Begießen des Alumosilikats erfolgen. Vorzugsweise erfolgt das Besprühen oder Begießen des Alumosilikats mit der Alkali- und/oder Magnesiumsalzlösung in dem die Lösung auf das in einer Trommel rotierende Alumosilikat gesprüht oder gegossen wird. Die Behandlung kann in einem Zug erfolgen, d. h. zum Alumosilikat wird zu Beginn die gesamte Menge an Alkali- und/oder Magnesiumsalzlösung in einem Schritt zugegeben. Die Salzlösung kann aber auch durch Besprühen oder Begießen in kleinen Portionen zudosiert werden, wobei der Zeitraum der Zugabe vorzugsweise von 0,1 bis 10 h und bevorzugt von 1 bis 3 h beträgt. Die Menge an Salzlösung wird vorzugsweise so bemessen, dass die gesamte Lösung vom Alumosilikat aufgenommen wird.

Insbesondere das Tränken aber auch das Besprühen bzw. Begießen kann in üblichen technischen Apparaturen, wie beispielsweise Konusmischern oder Intensivmischern, wie sie z. B. von der Firma Eirich angeboten werden, durchgeführt werden.

Die Behandlung des Alumosilikats mit der Alkali- und/oder Magnesiumsalzlösung in Schritt a) kann in einem Schritt oder in mehreren Teilschritten erfolgen. Insbesondere ist es möglich, die Behandlung in zwei oder mehreren Teilschritten durchzuführen. In jedem der einzelnen Teilschritte kann jeweils die gleiche Alkali- oder Magnesiumsalzlösung eingesetzt werden oder aber in jedem Teilschritt eine in Konzentration und/oder Zusammensetzung verschiedene Alkali- und/oder Magnesiumsalzlösung. Beispielsweise kann zum Alumosilikat zunächst nur ein Teil der Alkali- und/oder Magnesiumsalzlösung zugesetzt werden und, gegebenenfalls nach einer Zwischentrocknung, bei gleicher oder anderer Temperatur die Restmenge der eingesetzten Salzlösung. Es ist aber auch möglich die Behandlung des Alumosilikats mit unterschiedlichen Alkali- und/oder Magnesiumsalzlösungen (unterschiedliche Konzentration und/oder Zusammensetzung) bei gleicher oder unterschiedlicher Temperatur durchzuführen. Es ist nicht nur möglich, dass der Schritt a) in zwei oder mehreren Teilschritten durchgeführt wird. Ebenfalls ist es möglich, wenn das Verfahren mehrere Schritte a) aufweist. Auch in diesem Fall können in den verschiedenen Schritten a) gleiche oder unterschiedliche Alkali- und/oder Magnesiumsalzlösungen in Bezug auf Konzentration und/oder Zusammensetzung eingesetzt werden.

Das Behandeln in Schritt a) kann vorzugsweise bei einer Temperatur von 10 bis 120 °C, bevorzugt von 10 bis 90 °C, besonders bevorzugt von 15 bis 60 °C und ganz besonders bevorzugt bei einer Temperatur von 20 bis 40 °C durchgeführt werden.

Es kann vorteilhaft sein, wenn in Schritt a) ein oder mehrere Additive dem Alumosilikat bzw. der Alkali- und/oder Magnesiumsalzlösung zugegeben bzw. zugemischt werden. Solche Additive können z. B. Bindemittel, Gleitmittel oder Formgebungshilfsmittel sein. Ein geeignetes Bindemittel kann z. B. Boehmit oder Pesudo-Boehmit sein, wie es z. B. unter der Bezeichnung Disperal (einem Boehmit mit einem formalen Al₂O₃-Gehalt von ca. 77 Massen-%) von der Sasol Deutschland GmbH angeboten wird. Wird Boehmit, insbesondere Disperal als Bindemittel zugegeben, so wird dieses vorzugsweise als Gel zugegeben, welches z. B. durch Einrühren von 197 Massenteilen Disperal in 803 Massenteilen einer 1,28 Massen-%igen, wässrigen Salpetersäure, gründliches Rühren bei 60 °C für 3 h, Abkühlen auf Raumtemperatur und Ergänzen von evtl. verdunstetem Wasser erhalten werden kann. Als Formgebungshilfsmittel können beispielsweise Kieselsäuren, insbesondere pyrogene Kieselsäuren, wie sie z. B. von der Degussa AG unter der Bezeichnung Aerosil vertrieben werden, Bentonite, Tone, Kaolin, Kaolinit, ball clay und andere, dem Fachmann hierfür geläufige Stoffe eingesetzt werden. Als Gleitmittel, dessen Einsatz für die verbesserte Tablettierung vorteilhaft sein kann, kann beispielsweise Graphit zugesetzt werden.

Die Zugabe eines oder mehrerer der oben genannten Additive in Schritt a) kann auf verschiedene Weisen erfolgen. Insbesondere kann die Zugabe während der Behandlung des Alumosilikats mit der Alkali- und/oder Magnesiumsalzlösung erfolgen. Beispielweise können Alumosilikat, Additiv und Alkali- und/oder Magnesiumsalzlösung in eine technische Apparatur gefüllt und anschließend innig vermischt werden. Eine andere Möglichkeit besteht darin, zuerst das Alumosilikat mit dem Additiv zu vermischen und anschließend die Alkali-und/oder Magnesiumsalzlösung zuzusetzen. In einer weiteren Variante kann zum Alumosilikat Additiv und Alkali- und/oder Magnesiumsalzlösung gleichzeitig zudosiert werden. Die Zugabe kann jeweils in einem Guss, in Portionen oder durch Sprühen erfolgen. Die Zugabezeit beträgt vorzugsweise weniger als 5 h, bevorzugt weniger als 3 h. Es kann vorteilhaft sein, die Mischung für 0,1 bis 10 h, bevorzugt für 0,5 bis 3 h nachzumischen.

### Verfahrensschritt b):

Das erfindungsgemäße Verfahren weist zumindest einen Verfahrensschritt b) auf, bei dem das mit Alkali- und/oder Magnesiumsalzlösung behandelte Alumosilikat kalziniert wird. Die Kalzinierung erfolgt vorzugsweise in einem Gasstrom, beispielsweise in einem Gasstrom, der z. B. Luft, Stickstoff, Kohlendioxid und/oder eines oder mehrere Edelgase enthält oder aus einer oder mehrerer dieser Komponenten besteht. Bevorzugt erfolgt die Kalzinierung unter Verwendung von Luft als Gasstrom.

Die Kalzinierung im erfindungsgemäßen Verfahrensschritt b) wird vorzugsweise bei einer Temperatur von 200 bis 1000 °C, bevorzugt von 300 bis 800 °C durchgeführt. Die Kalzinierung erfolgt vorzugsweise für eine Dauer von 0,1 bis 10 Stunden, bevorzugt von 1 bis 5 Stunden. Besonders bevorzugt wird die Kalzinierung bei einer Temperatur von 200 bis 1000 °C, vorzugsweise 300 bis 800 °C für 0,1 bis 10 Stunden, bevorzugt von 1 bis 5 Stunden durchgeführt.

Bevorzugt kann die technische Kalzinierung in einem Schachtofen durchgeführt werden. Die Kalzinierung kann jedoch auch in anderen bekannten technischen Apparaturen durchgeführt werden, wie beispielsweise Wirbelschichtkalzinierern, Drehrohröfen oder Hordenöfen.

### Verfahrensschritt c):

Erfindungsgemäß wird zwischen den Schritten a) und b) ein Schritt c) durchgeführt in dem das mit Magnesiumsalzlösung behandelte Alumosilikat getrocknet wird. Die Trocknung in Schritt c) kann bei einer Temperatur von 100 bis 140 °C erfolgt. Vorzugsweise erfolgt die Trocknung in einem Gasstrom. Die Trocknung kann beispielsweise in einem Gasstrom, der z. B. Luft, Stickstoff, Kohlendioxid und/oder eines oder mehrere Edelgase enthält oder aus einer oder mehrerer dieser Komponenten besteht, durchgeführt werden. Durch den Zwischenschritt des Trocknens nach der Behandlung mit Magnesiumsalzlösung und vor dem Kalzinieren wird erreicht, dass bei der Kalzinierung keine großen Wasserdampfmengen freigesetzt werden. Zudem kann durch das Trocknen verhindert werden, dass durch beim Kalzinieren spontan verdampfendes Wasser die Form des Katalysators zerstört.

Je nachdem in welcher gewünschten Form der Katalysator vorliegen soll, kann es vorteilhaft sein, das Herstellungsverfahren durch zusätzliche Verfahrensschritte entsprechend anzupassen.

Die Katalysatorformkörper liegen in Form von Tabletten vor. Um zum Formgegebenen Katalysator (Katalysatorformkörper) zu gelangen, können abhängig von der jeweiligen Formgebungsvariante neben den Verfahrensschritten Behandlung, Trocknung, Kalzination, weitere Verfahrensschritte, wie z. B. Formgebung, Mahlung oder Siebung, durchgeführt werden. Formgebungshilfsmittel können an verschiedenen Stellen im Prozess eingebracht werden. Die Herstellung der Katalysatorformkörper kann auf unterschiedliche Weise erfolgen:

In der Ausführungsform des erfindungsgemäßen Verfahrens können Tabletten des Katalysators, insbesondere des erfindungsgemäßen Katalysators, dadurch erhalten werden, dass in Verfahrensschritt a) ein Alumosilikatpulver, Bindemittel, optional Gleitmittel und saure wässrige Alkali- und/oder Erdalkalimetallsalzlösung gemischt werden und das so behandelte Alumosilikatpulver, z. B. in einem Eirichmischer, zu Mikropellets vorzugsweise mit einem mittleren Durchmesser 0,5 bis 10 mm, bevorzugt 1 bis 5 mm und besonders bevorzugt von 1 bis 3 mm (Bestimmung der Korngröße kann z. B. durch Siebanalyse erfolgen) pelletiert wird und die erhaltenen feuchten Pellets in Verfahrensschritt c) getrocknet und anschließend optional in Verfahrensschritt b) im Gasstrom kalziniert werden. Die erhaltenen Pellets können dann, falls in Verfahrensschritt a) noch nicht geschehen mit einem Gleitmittel, wie z. B. Graphit, vermischt und anschließend auf einer handelsüblichen Tablettenpresse, z. B. einer Rundläuferpresse, tablettiert werden. Die Tabletten können dann, falls noch kein Verfahrensschritt b) durchgeführt wurde, in Verfahrensschritt b) kalziniert werden oder optional nachkalziniert werden.

Mit dem erfindungsgemäßen Verfahren sind insbesondere Katalysatoren erhältlich, die formal aus Aluminiumoxid (Al₂O₃) und Siliziumdioxid (SiO₂) und mindestens einem Oxid aus der Gruppe der Alkali- und Magnesiumoxid bestehen. Der durchschnittliche Massenanteil an Aluminiumoxid beträgt bei diesen Katalysatoren von 4 bis 20 %, der an Siliziumdioxid von 70 bis 90 % und der Massenanteil der Alkali- und Erdalkalimetalloxide in Summe vorzugsweise von 0.5 bis 20 %. Erfingsgemäß weisen solche Katalysatoren einziges Erdalkalimetall Magnesium auf.

Der Katalysator, der formal Alkalimetall- und Magnesiumoxid, Aluminiumoxid und Siliziumdioxid aufweist, zeichnet sich dadurch aus, dass er formal einen Anteil an Alkalimetall- und Magnesiumoxid (Summe der im Katalysator enthaltenen Alkali- und Magnesiumoxid) von 0,5 bis 20 Massen-%, einen Anteil an Aluminiumoxid (Al₂O₃) von 4 bis 20 Massen-%, und einen Anteil an Siliziumdioxid von 70 bis 90 Massen-% aufweist. Der Katalysator kann z. B. durch das oben beschriebene erfindungsgemäße Verfahren erhalten werden.

Der Katalysator enthält Magnesiumoxid. Er enthält als einziges Erdalkalimetalloxid Magnesiumoxid. Es ist vorteilhaft, dass der Katalysator neben dem Magnesiumloxid ein Alkalimetalloxid aufweist. Dieses ist ausgewählt aus Na₂O oder K₂O. Vorzugsweise weist der erfindungsgemäße Katalysator als Alkalimetalloxid Na₂O auf.

Der Katalysator weist vorzugsweise einen Anteil an Magnesiumoxid von 5 bis 15 Massen-% und besonders bevorzugt von 10 bis 15 Massen-%, einen Anteil an Aluminiumoxid von 4 bis 20 Massen-%, bevorzugt von 10 bis 20 Massen-% und einen Anteil an Siliziumdioxid von 70 bis 90 Massen-% auf.

Der Katalysator weist vorzugsweise eine BET-Oberfläche (volumetrisch bestimmt mit Stickstoff gemäß DIN ISO 9277) von 200 bis 450 m²/g, bevorzugt von 200 bis 350 m²/g auf. Wird der Katalysator als aktive Masse auf einem Träger aufgebracht, so weist nur die aktive Masse eine BET-Oberfläche im genannten Bereich auf. Das Material aus Katalysator und Träger kann dagegen je nach Beschaffenheit des Trägers eine deutlich abweichende BET-Oberfläche, insbesondere eine kleinere BET-Oberfläche aufweisen. Das Porenvolumen des Katalysators beträgt vorzugsweise von 0,5 bis 1,3 ml/g, bevorzugt von 0,65 bis 1,1 mg. Das Porenvolumen wird vorzugsweise bestimmt durch die Cyclohexanmethode.

Bei dieser Methode wird die zu prüfende Probe zunächst bei 110 °C bis zur Gewichtskonstanz getrocknet. Anschließend werden ca. 50 ml der auf 0,01 g genau eingewogenen Probe in ein gereinigtes und bis zur Gewichtskonstanz getrocknetes Imprägnierrohr gefüllt, dass an der Unterseite eine Auslauföffnung mit einem Schliffhahn aufweist. Die Auslauföffnung ist mit einem kleinen Plättchen aus Polyethylen abgedeckt, wodurch ein Verstopfen der Auslauföffnung durch die Probe verhindert wird Nach dem Befüllen des Imprägnierrohres mit der Probe wird das Rohr sorgfältig luftdicht verschlossen. Anschließend wird das Imprägnierrohr mit einer Wasserstrahlpumpe verbunden, der Schliffhahn geöffnet und über den Wasserstrahl ein Vakuum im Imprägnierrohr von 20 mbar. eingestellt. Das Vakuum kann an einem parallel angeschlossenen Vakuummeter geprüft werden. Nach 20 min. wird Schliffhahn geschlossen und das evakuierte Imprägnierrohr wird anschließend so mit einer Cyclohexanvorlage, in der ein genau abgemessenes Volumen an Cyclohexan vorgelegt wird, verbunden, dass durch Öffnen des Schliffhahns Cyclohexan aus der Vorlage in das Imprägnierrohr gesaugt wird. Der Schliffhahn bleibt so lange geöffnet, bis die gesamte Probe mit Cyclohexan geflutet ist. Anschließend wird der Schliffhahn wieder verschlossen. Nach 15 min wird das Imprägnierrohr vorsichtig belüftet und das nicht absorbierte Cyclohexan in die Vorlage abgelassen. Im Imprägnierrohr bzw. in der Auslauföffnung oder der Verbindung mit der Cyclohexanvorlage anhaftendes Cyclohexan kann durch einen einzelnen vorsichtigen Druckstoß aus einem Saugball, über die Belüftungsleitung in die Vorlage befördert werden. Das Volumen des in der Vorlage vorhandenen Cyclohexans wird notiert. Das Porenvolumen ergibt sich aus dem absorbierten Cyclohexanvolumen, welches bestimmt wird aus dem Cyclohexanvolumen in der Vorlage vor der Messung minus dem Cyclohexanvolumen in der Vorlage nach der Messung, geteilt durch die Masse der untersuchten Probe.

Der mittlere Porendurchmesser (vorzugsweise bestimmt in Anlehnung an DIN 66133) des Katalysators beträgt vorzugsweise von 5 bis 20 nm, bevorzugt von 8 bis 15 nm. Besonders bevorzugt entfällt mindestens 50 %, vorzugsweise über 70 % des Gesamtporenvolumens (Summe des Porenvolumens der Poren mit einem Porendurchmesser von größer-gleich 3,5 nm bestimmt mit Quecksilberporosimetrie gemäß DIN 66133) des Katalysators auf Poren mit einem Durchmesser von 3,5 bis 50 nm (Mesoporen).

Die Katalysatoren können unterschiedliche Abmessungen, insbesondere eine Abmessung von 10 µm bis 10 mm aufweisen. Für den Einsatz des Katalysators in Festbettreaktoren, liegt dieser vorzugsweise als Tablette, vor und weist vorzugsweise Abmessungen (längste Dimension oder Durchmesser) von 0,5 bis 10 mm, bevorzugt von 1 bis 5 mm auf. Erfindungsgemäß wird mit dem Katalysator Methyl-tert.-butylether in Isobuten und Methanol gespalten.

Eingesetzt werden können in dem Spaltverfahren ATAE, die aus den unterschiedlichsten Prozessen stammen können. Ein Verfahren zur Herstellung von MTBE wird beispielsweise in DE 101 02 062 beschrieben. Verfahren zur Herstellung von ETBE werden beispielsweise in DE 10 2005 062700, DE 10 2005 062722, DE 10 2005 062699 oder DE 10 2006 003492 offen gelegt.

Die Spaltung in der Gasphase am erfindungsgemäß hergestellten Katalysator wird vorzugsweise bei einer Temperatur von 150 bis 400 °C durchgeführt. Wird als Ausgangsprodukt MTBE eingesetzt, wird die Spaltung von MTBE zu Isobuten und Methanol bevorzugt bei einer Temperatur von 180 bis 400 °C, besonders bevorzugt von 230 bis 350 °C durchgeführt.

Das Spaltverfahren wird vorzugsweise bei einem Reaktionsdruck von 0,1 bis 1 MPa durchgeführt. Wenn Isobuten ein Produkt ist kann es vorteilhaft sein, das Spaltverfahren bei einem Druck von 0,2 bis 1 MPa, bevorzugt von 0,5 bis 0,8 MPa durchzuführen. Dies ist insbesondere deshalb vorteilhaft, weil bei diesen Drücken Isobuten gegen Kühlwasser kondensiert werden kann.

Die spezifische Katalysatorbelastung (WHSV; Gramm Edukt bei Raumtemperatur je Gramm Katalysator je Stunde) beträgt im Spaltverfahren vorzugsweise von 0,1 bis 100 h⁻¹, bevorzugt von 0,5 bis 30 h⁻¹. Wird als Ausgangsprodukt MTBE eingesetzt, wird die Spaltung von MTBE zu Isobuten und Methanol bevorzugt bei einer WHSV von 0,1 bis 100 h⁻¹, besonders bevorzugt von 0,25 bis 25 h⁻¹ durchgeführt.

Um den Aufarbeitungsaufwand des Spaltproduktgemisches gering zu halten, werden vorzugsweise hohe Umsätze für den geraden Durchgang angestrebt. Vorzugsweise wird das Verfahren so durchgeführt, dass die Umsätze an zu spaltender Verbindung über 70 %, bevorzugt über 80 % und besonders bevorzugt von über 90 % bis 100 % betragen.

Wenn die Edukte störende Nebenkomponenten enthalten, kann es zweckmäßig sein, den Umsatz zu begrenzen. Enthält beispielsweise das Einsatzstoffgemisch neben dem zu spaltenden MTBE auch 2-Methoxybutan, kann es notwendig sein, den Umsatz im geraden Durchgang zu verringern, um ein vorgegebenes Verhältnis von linearen Butenen zu Isobuten im Reaktionsgemisch nicht zu überschreiten. Somit kann es vorteilhaft sein, den zulässigen Umsatz an MTBE zu begrenzen, je höher der Anteil an 2-Methoxybutan im MTBE aufweisenden Einsatzstoffgemisch ist. Die Begrenzung des Umsatzes kann beispielsweise durch Erhöhung der WHSV und/oder Absenken der Reaktionstemperatur erreicht werden.

Die Selektivität der Isoolefin-Bildung beträgt bei dem Verfahren vorzugsweise über 98 %, bevorzugt über 99 %. Die Selektivität für die Bildung von Alkoholen beträgt bei dem Verfahren bei der ATAE-Spaltung, insbesondere bei der ATBE Spaltung über 95 %, insbesondere über 99 %.

Das Spaltproduktgemisch kann nach bekannten technischen Verfahren aufgearbeitet werden. Nicht umgesetztes Edukt kann gegebenenfalls nach einer Teilausschleusung oder Reinigung in die Spaltung zurückgeführt werden.

Die gewonnenen Isoolefine können, wie in der Einleitung beschrieben, genutzt werden. Ein gemäß dem erfindungsgemäßen Spaltverfahren hergestelltes Isobuten kann insbesondere zur Herstellung von Butylkautschuk, Polyisobutylen, Isobuten-Oligomeren, verzweigten C₅-Aldehyden, C₅-Carbonsäuren, C₅-Alkoholen, C₅-Olefinen, tert.-Butylaromaten und Methacrylsäure und deren Ester eingesetzt werden.

Die bei der Spaltung von MTBE anfallendes Methanol kann nach Anforbeitung wieder verwendet werden, z.B. zur Synthese von MTBE.

Die folgenden Beispiele sollen das Verfahren erläutern.

### Beispiel 1: Herstellung eines Alumosilikatformkörpers

500 g Alumosilikatpulver (Hersteller: Grace Davison, Typ: Davicat O 701, formaler Al₂O₃-Gehalt: 13 Massen-%, formaler SiO₂-Gehalt: 76 Massen-%, formaler Na₂O-Gehalt: 0,1 Massen-%, Glühverlust bei 850 °C: ca. 11 %), 363 g Disperal-Gel (formaler Al₂O₃-Gehalt: 15,6 %), welches durch Einrühren von 197 g Disperal, einem Boehmit mit einem formalen Al₂O₃-Gehalt von 77 Massen-%, der Sasol Deutschland GmbH in 803 g einer 1,28 Massen-%igen, wässrigen Salpetersäure, anschließendes gründliches Rühren, bei dem das sich bildende Gel ständig geschert und so in einem fließfähigen Zustand gehalten wird, in einem abgedeckten Behälter für 3 h bei 60 °C, Abkühlen des Gels auf Raumtemperatur und Ersetzen von eventuell verdunstetem

Wasser erhalten wird, und 370 g vollentsalztes Wasser (VE-Wasser) wurden zunächst gründlich miteinander in einem Intensiv-Mischer der Firma Eirich vermischt. Anschließend erfolgte eine Pelletierung in dem Intensiv-Mischer der Firma Eirich, bei der innerhalb von 30-40 Minuten gleichmäßig rundliche Pellets mit einem Durchmesser von ca. 1 bis 3 mm erhalten wurden. Die feuchten Pellets wurden zunächst bei 120 °C im Luftstrom getrocknet und anschließend mit 2 K/min auf 550 °C aufgeheizt und bei dieser Temperatur für 10 h im Luftstrom kalziniert. Die so hergestellten Alumosilikatpellets enthielten formal 76 Massen-% Al₂O₃ und 24 Massen-% SiO₂. Weiterhin enthielt der hergestellte Katalysator 0,12 Massen-% Natriumverbindungen (berechnet als Natriumoxid). Die Zusammensetzung der Alumosilikatpellets wurde berechnet aus der Menge und der Zusammensetzung der Ausgangssubstanzen. Die Alumosilikatpellets wiesen ein Porenvolumen, bestimmt mit der oben beschriebenen Cyclohexanmethode, von 1,15 ml/g auf.

### Beispiel 2: Herstellung eines geformten Katalysators (nicht gemäß der Erfindung)

Aus VE-Wasser und Magnesiumnitrat-Hexahydrat wurde eine Imprägnierlösung mit einem Magnesiumgehalt von 4,7 Massen-% hergestellt. Der pH-Wert dieser Lösung betrug 5,1. Über eine Vakuumimprägnierung wurden eine ausgesiebte Fraktion des in Beispiel 1 hergestellten Alumosilikatträgers (Durchmesser: 1,0 mm - 2,8 mm) mit der sauren Magnesiumnitratlösung getränkt. Dazu wurden die Pellets in ein Glasrohr gefüllt und dieses für etwa 30 min evakuiert (Wasserstrahlpumpenvakuum von ca. 25 hPa). Anschließend wurde die Imprägnierlösung von unten bis über den oberen Rand der Festkörperschüttung gesaugt. Nach einer Einwirkzeit von etwa 15 Minuten wurde die Lösung, die nicht von dem Träger aufgenommen worden war, abgelassen. Die feuchten Pellets wurden zunächst im Luftstrom bei 140 °C bis zur Gewichtskonstanz getrocknet und anschließend mit 3 K/min auf 450 °C aufgeheizt und bei dieser Temperatur für 12 h kalziniert. Der hergestellte Katalysator bestand formal aus 68 Massen-% Siliziumdioxid, aus 21 Massen-% Aluminiumoxid und aus 11 Massen-% Magnesiumoxid. Weiterhin enthielt der hergestellte Katalysator 0,11 Massen-% Natriumverbindungen (berechnet als Natriumoxid). Die Zusammensetzung des Katalysators wurde berechnet aus der Menge und der Zusammensetzung der Ausgangssubstanzen sowie der abgelaufenen Imprägnierlösung. Die Natriummengen waren Bestandteil des in Beispiel 1 eingesetzten Alumosilikats. Das Porenvolumen, bestimmt mit der oben beschrieben Cyclohexanmethode, betrug 1,1 ml/g.

### Beispiel 3: Herstellung eines pulverförmigen MgO/Al₂O₃/SiO₂-Katalysators (durch Tränken eines Alumosilikats mit einer Magnesiumsalzlösung) (nicht gemäß der Erfindung)

500 g eines Alumosilicatpulvers (Hersteller: Grace Davison, Typ: Davicat O 701, Al₂O₃-Gehalt: 13 Gew. %, SiO₂-Gehalt: 76 Gew. %, Na₂O-Gehalt: 0,1 %, Glühverlust bei 850 °C: 11 %, Korngröße d₅₀ = 20 µm, bestimmt über Laserbeugung an einem Mastersizer 2000, der Firma Malvern) mit einem Porenvolumen von 1,1 ml/g wurden in einem Konusmischer mit 550 ml einer sauren Magnesiumnitratlösung (Mg-Gehalt: 4,7 Massen-%, pH-Wert = 5,1) versetzt und für 20 Minuten gemischt. Das feuchte Pulver wurde anschließend in einem Wirbelschichtofen zunächst bei 140 °C im Luftstrom bis zur Gewichtskonstanz getrocknet und anschließend bei 450 °C in einem Muffelofen für 10 h kalziniert. Der aus den Ausgangsverbindungen berechnete Gehalt der Hauptkomponenten (bezogen auf diese Hauptkomponenten) im bei 850 °C geglühten Katalysator betrug 12 Massen-% Al₂O₃, 77 Massen-% SiO₂ 11 Massen-% MgO.

### Beispiel 4: Herstellung von Katalysatortabletten (erfindungsgemäß)

In 355 g VE-Wasser wurden 645 g Magnesiumnitrat-Hexahydrat gelöst. Der pH-Wert dieser Lösung betrug 3,5. In diese Lösung wurden 500 g Disperal-Gel (formaler Al₂O₃-Gehalt: 15,6 Gew.-%) eingerührt. Die erhaltene viskose, aber noch gut rühr- und pumpbare Suspension wurde anschließend direkt mit 917 g Alumosilikatpulver (Hersteller: Grace Davison, Typ: Davicat O 701, Al₂O₃-Gehalt: 13 Gew. %, SiO₂-Gehalt: 76 Gew. %, Na₂O-Gehalt: 0,1 %, Glühverlust bei 850 °C: 11 %, Korngröße d₅₀ = 20 µm, bestimmt über Laserbeugung an einem Mastersizer 2000, der Firma Malvern) und 42 g Graphit (Hersteller: Edelgraphitgesellschaft Bonn, Typ: K 16) als Tablettierhilfsmittel vorgemischt. Dabei wurde eine Masse erhalten, die aus lockeren leicht feuchten Agglomeraten besteht. Diese Masse wurde anschließend in einem Intensiv-Mischer der Firma Eirich pelletiert. Um tablettierfähiges Granulat zu erhalten, wurde die Pelletierung dabei so gesteuert, dass überwiegend rundliche Pellets mit einem Durchmesser von kleiner als 1 mm entstanden. Die feuchten Pellets wurden anschließend für 2 h bei 140 °C angetrocknet und danach bei 450 °C in einem mit Luft durchströmten Drehrohr für 1 h kalziniert.

Die kalzinierten Pellets wurden anschließend mit einer Tablettenpresse (Firma: Ronchi) zu Tabletten mit einem Durchmesser von 3 mm und einer Höhe von 4 mm verpresst. Anschließend wurden die Tabletten bei 560 °C für 2 Stunden nachkalziniert. Der nominale Gehalt in der Tablette betrug an Al₂O₃ 19,7 Massenteile, an SiO_{2,} 70,8 Massenteile, an MgO 10,0 Massenteile, an Na₂O 0,1 Massenteile. Die Tabletten wiesen eine BET-Oberfläche (gemäß DIN ISO 9277) von 253 m²/g auf. Das Gesamtporenvolumen ermittelt mittels QuecksilberPorosimetrie in Anlehnung an DIN 66133 (maximaler Meßdruck: 400 MPa) für Poren mit einem Durchmesser von 3,5 bis 10000 nm betrug 0,68 ml/g. Das Makroporenvolumen (Poren mit Durchmesser von 50-10000 nm) betrug dabei 0,1 ml/g (Makroporenanteil 15 %) und das Mesoporenvolumen (Poren mit Durchmesser von 3,5 bis 50 nm) betrug 0,57 ml/g (Mesoporenanteil 85 %). Der mittlere Porendurchmesser betrug 9,6 nm.

### Beispiel 5: Gasphasenspaltung von MTBE zu Isobuten und Methanol (nicht erfindungsgemäß)

Die Spaltung wurde in einem Rohrreaktor mit Heizmantel, durch den ein Wärmeträgeröl (Marlotherm SH der Sasol Olefins & Surfactants GmbH) floss, durchgeführt. Als Katalysator wurde der im Beispiel 2 hergestellte Katalysator verwendet. Als Edukt wurde technisches MTBE (Driveron der Fa. Oxeno Olefinchemie GmbH) mit einer Reinheit von 99,7 Massen- % eingesetzt.

Vor dem Eintritt in den Reaktor wurde das MTBE in einem Verdampfer bei 250 °C vollständig verdampft. Bei einer Temperatur von 250 °C (Temperatur des Marlotherms im Zulauf des Reaktormantels) und einem Druck von 7 bar_{absolut} (bara) wurden stündlich 500 g MTBE durch 282 g Katalysator, entsprechend einem WHSV-Wert von 1,77 h⁻¹, durchgeleitet. Das gasförmige Spaltproduktgemisch wurde in einem Kühler teilkondensiert. Die erhaltene flüssige Phase wurde ausgewogen und die Gasphase ausgelitert. Beide Phasen wurden gaschromatographisch analysiert.

Die Spaltung wurde unter den obigen Bedingungen über einen Zeitraum von mehr als 2500 Stunden durchgeführt. Aus der Zusammensetzung des Spaltproduktgemisches, der Eduktmasse und der Masse des Spaltproduktgemisches wurden die Isobuten-Umsätze, die Selektivitäten der Isobutenbildung (Molzahl des gebildeten Isobutens zu Molzahl des umgesetzten MTBE) und die Selektivitäten der Methanolbildung (Molzahl des gebildeten Methanols zu Molzahl des umgesetzten Isobutens) bei verschiedenen Reaktionszeiten berechnet. Die erhaltenen Werte wurden in der nachfolgenden Tabelle 1 zusammengestellt.

**Tabelle 1: Umsätze und Selektivitäten der Spaltung von MTBE gemäß Beispiel 5**

| Versuchsdauer (h) | 51 | 99 | 495 | 1000 | 1511 | 1992 | 2497 |
|---|---|---|---|---|---|---|---|
| MTBE-Umsatz (%) | 85,4 | 84,2 | 81,1 | 77,3 | 76,2 | 73,6 | 72,5 |
| Isobuten-Selektivität (%) | 99,98 | 99,98 | 99,97 | 99,97 | 99,97 | 99,97 | 99,97 |
| Methanol-Selektivität (%) | 97,1 | 97,4 | 97,8 | 97,8 | 97,7 | 97,8 | 97,7 |

Der Versuch zeigt, dass der erfindungsgemäße Katalysator sehr gut für die Spaltung von MTBE zu Isobuten und Methanol geeignet ist. Die Selektivitäten sowohl der Isobutenbildung als auch der Methanolbildung waren ausgezeichnet und blieben während der gesamten Versuchsdauer nahezu konstant. Die Aktivität des Katalysators nahm mit zunehmender Versuchsdauer ab. Diese betrug nach ca. 2500 Stunden jedoch immer noch 85 % der Aktivität des Katalysators nach 50 h Versuchsdauer, sodass bei einer technischen Produktionsanlage von einer Standdauer des Katalysators von weit über einem Jahr ausgegangen werden kann.

### Beispiel 6: Gasphasenspaltung von MTBE zu Isobuten und Methanol (erfindungsgemäß)

In der gleichen Apparatur wie in Beispiel 5 wurde MTBE bei 250 °C gespalten. Als Katalysator wurden 10 g der in Beispiel 4 hergestellten Tabletten verwendet. Der Durchsatz betrug 100 g MTBE je Stunde. Dies entsprach einer WHSV von 10 h⁻¹. Der Versuch lief über 480 Stunden. Die Versuchsergebnisse wurden in Tabelle 2 zusammengestellt.

**Tabelle 2: Umsätze und Selektivitäten der Spaltung von MTBE gemäß Beispiel 6**

| Versuchsdauer (h) | 48 | 144 | 480 |
|---|---|---|---|
| MTBE-Umsatz (%) | 86,9 | 80,2 | 67,4 |
| Isobuten-Selektivität (%) | 99,7 | 99,8 | 99,9 |
| Methanol-Selektivität (%) | 99,27 | 99,29 | 99,12 |

Der Versuch zeigt, dass der tablettierte Katalysator aus Beispiel 4 eine höhere Aktivität als der Katalysator aus Beispiel 2 aufweist. Die Selektivität der Methanolbildung ist besser und die Isobutenbildung ist etwas geringer als beim Katalysator aus Beispiel 2, jedoch weiterhin hervorragend. Auch dieser Katalysator eignet sich für die technische Herstellung von Isobuten durch Spaltung von MTBE. Wegen der hohen Aktivität des Katalysators ist dabei nur ein relativ geringes Katalysatorvolumen erforderlich.

## Patentansprüche

1. Verfahren zur Herstellung eines Katalysators zur Gasphasenspaltung von MTBE (Methyl-tert.-butylether) in Isobuten und Methanol, der formal
• einen Anteil an Alkali- und Erdalkalimetalloxiden von 0,5 bis 20 Massen-%,
• einen Anteil an Aluminiumoxid von 4 - 20 Massen-%
• und einen Anteil an Siliziumdioxid von 70 - 90 Massen-%
aufweist;
wobei der Katalysator als einziges Erdalkalimetalloxid Magnesiumoxid enthält;
wobei der Katalysator neben dem Erdalkalimetalloxid ein Alkalimetalloxid aufweist, welches ausgewählt ist aus Na₂O und K₂O,
welches die Schritte
a) Behandeln eines Alumosilikats mit einer wässrigen Magnesiumsalzlösung unter sauren Bedingungen und
b) Kalzinieren des mit wässriger Magnesiumsalzlösung behandelten Alumosilikats aufweist,
bei welchem zwischen den Schritten a) und b) ein Schritt c) durchgeführt wird, in dem das behandelte Alumosilikat getrocknet wird,
wobei der Katalysator dadurch erhalten wird, dass in Verfahrensschritt a) ein Alumosilikatpulver, Bindemittel, optional Gleitmittel und saure wässrige Magnesiumsalzlösung gemischt werden und das so behandelte Alumosilikatpulver zu Mikropellets pelletiert wird und die erhaltenen feuchten Pellets in Verfahrensschritt c) getrocknet und anschließend in Verfahrensschritt b) im Gasstrom kalziniert werden, die erhaltenen Pellets dann, falls in Verfahrensschritt a) noch nicht geschehen mit einem Gleitmittel, wie z. B. Graphit, vermischt und anschließend tablettiert werden und die Tabletten dann nachkalziniert werden.

2. Verfahren nach Anspruch 1,
**dadurch gekennzeichnet,**
**dass** die Trocknung in Schritt c) bei einer Temperatur von 100 bis 140 °C erfolgt.

3. Verfahren nach zumindest einem der Ansprüche 1 bis 2.
**dadurch gekennzeichnet,**
**dass** das Behandeln in Schritt a) bei einer Temperatur von 10 bis 120 °C durchgeführt wird.

4. Verfahren nach zumindest einem der Ansprüche 1 bis 3,
**dadurch gekennzeichnet,**
**dass** die Kalzinierung in Schritt b) bei einer Temperatur von 200 bis 1000 °C für 0,1 bis 10 Stunden durchgeführt wird.

5. Verfahren nach Anspruch 4,
**dadurch gekennzeichnet,**
**dass** eine Magnesiumnitrat-Lösung eingesetzt wird.

## Claims

1. A process for preparing a catalyst for gas phase cleavage of MTBE (methyl tert-butyl ether) to isobutene and methanol, said catalyst, in a formal sense, having
• a content of alkali metal and alkaline earth metal oxides of 0.5 to 20% by mass,
• a content of aluminium oxide of 4 to 20% by mass
• and a content of silicon dioxide of 70 to 90% by mass,
the catalyst containing magnesium oxide as the sole alkaline earth metal oxide,
and the catalyst comprising, in addition to the alkaline earth metal oxide, an alkali metal oxide selected from Na₂O and K₂O,
which comprises the steps of
a) treating an aluminosilicate with an aqueous magnesium salt solution under acidic conditions and
b) calcining the aluminosilicate treated with aqueous magnesium salt solution,
in which a step c) in which the treated aluminosilicate is dried is performed between steps a) and b),
the catalyst being obtained by, in process step a), mixing an aluminosilicate powder, binder, optionally lubricant and acidic aqueous magnesium salt solution, and pelletizing the aluminosilicate powder thus treated to give micropellets, and drying the resulting moist pellets in process step c) and then optionally calcining them in a gas stream in process step b), then mixing the resulting pellets, unless already done in process step a), with a lubricant, for example graphite, and subsequently tabletting them and then post-calcining the tablets.

2. A process according to Claim 1,
**characterized in that**
the drying in step c) is effected at a temperature of 100 to 140°C.

3. A process according to at least one of Claims 1 and 2,
**characterized in that**
the treatment in step a) is performed at a temperature of 10 to 120°C.

4. A process according to at least one of Claims 1 to 3,
**characterized in that**
the calcination in step b) is performed at a temperature of 200 to 1000°C for 0.1 to 10 hours.

5. A process according to Claim 4,
**characterized in that**
a magnesium nitrate solution is used.

## Revendications

1. Procédé pour la préparation d'un catalyseur destiné à la dissociation en phase gazeuse de MTBE (méthyl-tert-butyléther) en isobutène et en méthanol, qui présente formellement
- une proportion d'oxydes de métal alcalin et alcalino-terreux de 0,5 à 20% en masse,
- une proportion d'oxyde d'aluminium de 4 à 20% en masse
- et une proportion de dioxyde de silicium de 70 à 90% en masse
le catalyseur contenant, comme unique oxyde de métal alcalino-terreux, de l'oxyde de magnésium ;
le catalyseur présentant, outre l'oxyde de métal alcalino-terreux, un oxyde de métal alcalin, qui est choisi parmi Na₂O et K₂O,
qui présente les étapes
a) de traitement d'un aluminosilicate par une solution aqueuse de sel de magnésium dans des conditions acides et
b) de calcination de l'aluminosilicate traité par une solution aqueuse de sel de magnésium,
dans lequel une étape c) est réalisée entre les étapes a) et b), lors de laquelle l'aluminosilicate traité est séché,
le catalyseur étant obtenu en ce que, dans l'étape de procédé a), une poudre d'aluminosilicate, un liant, éventuellement un lubrifiant et une solution aqueuse acide de sel de magnésium sont mélangés, la poudre d'aluminosilicate ainsi traitée est pelletisée en micropellets et les pellets humides obtenus sont séchés dans l'étape de procédé c) et ensuite calcinés dans l'étape de procédé b) dans un flux gazeux, les pellets obtenus sont ensuite mélangés, si cela n'a pas encore eu lieu dans l'étape de procédé a), avec un lubrifiant, tel que par exemple le graphite, puis transformés en comprimés et les comprimés sont ensuite post-calcinés.

2. Procédé selon la revendication 1, **caractérisé en ce que** le séchage dans l'étape c) a lieu à une température de 100 à 140°C.

3. Procédé selon au moins l'une quelconque des revendications 1 à 2, **caractérisé en ce que** le traitement dans l'étape a) est réalisé à une température de 10 à 120°C.

4. Procédé selon au moins l'une quelconque des revendications 1 à 3, **caractérisé en ce que** la calcination dans l'étape b) est réalisée à une température de 200 à 1000°C pendant 0,1 à 10 heures.

5. Procédé selon la revendication 4, **caractérisé en ce qu'**on utilise une solution de nitrate de magnésium.
